# EUROPEAN PATENT APPLICATION

(11) **EP 4 763 868 A1**
(43) Date of publication of application: **24.06.2026**
(21) Application number: 25772911.1
(22) Date of filing: 07.03.2025
(51) Int. Cl.: C07K 19/00, C12N 15/62, C12N 15/70, A61K 38/29, A61K 45/06, A61P 5/14

(54) **RECOMBINANT PTH FUSION PROTEIN, CONSTRUCTION METHOD THEREFOR, AND APPLICATION THEREOF**

(30) Priority: 19.03.2024 CN 202410309106
(71) Applicant: KANGZHONG (GUANGDONG) INC., Guangdong 528445 (CN); Sysvax Inc., Zhongshan, Guangdong 528400 (CN)
(72) Inventor: LI, Yong, Zhongshan, Guangdong 528400 (CN); GUO, Lijun, Zhongshan, Guangdong 528400 (CN); ZHUANG, Wenchao, Zhongshan, Guangdong 528400 (CN); CUI, Yingjie, Zhongshan, Guangdong 528400 (CN); LIN, Zhaoxin, Zhongshan, Guangdong 528400 (CN)
(74) Representative: Bottino, Giovanni
(86) International application number: PCT/CN2025/081281
(87) International publication number: WO 2025/195203

(57) **Abstract**

A recombinant PTH fusion protein, a construction method therefor, and an application thereof. The recombinant PTH fusion protein comprises: A1) at least one signaling domain molecule capable of activating a PTHR1 intracellular pathway; A2) a molecule having one or more ECDs capable of binding to the PTHR1; A3) a human serum albumin nanobody, wherein an amino acid sequence of the human serum albumin nanobody is as shown in SEQ ID NO: 7. The recombinant PTH fusion protein is capable of effectively improving the duration and magnitude of pharmacodynamic action, increasing blood calcium levels within 24-48 hours, and returning to normal levels within 48-72 hours. Said protein has excellent temperature stability and freeze-thaw stability, and can be used for treating and/or preventing hypoparathyroidism. In addition, the preparation method for the recombinant PTH fusion protein is simple and can be used for industrial production.

## Description

### TECHNICAL FIELD

The present disclosure relates to the technical field of protein engineering, and in particular, to a recombinant PTH fusion protein, a construction method therefor and use thereof.

### BACKGROUND

Hypoparathyroidism (HP) is a disease characterized by low blood calcium levels and elevated phosphate levels caused by insufficient circulating concentrations of parathyroid hormone (PTH). The symptoms of this disease are caused by increased neuromuscular irritability due to hypocalcemia, including tingling, muscle spasms, and seizures. The most common cause of HP is the accidental removal or injury of the parathyroid glands during neck surgery, followed by genetic, idiopathic, and autoimmune etiologies. According to the report "Hypoparathyroidism Market Insights, Epidemiology, and Market Forecasts 2032" commercially released by DelveInsight on November 7, 2022, as of 2021, the number of hypoparathyroidism patients in 7 major countries in Europe and America exceeded 280,200 (with 80,000 new cases), including more than 81,300 in the United States, more than 34,500 in five European countries, and more than 22,300 in Japan. The report further predicts a compound annual growth rate as high as 17.7% for the next decade from 2023 to 2032. There is no comprehensive epidemiological data on hypoparathyroidism in China, but based on relevant literature and foreign epidemiological data estimates, the number of HP patients in China is currently about 400,000.

The clinical manifestations of hypoparathyroidism are diverse and can involve almost any organ system, including epilepsy, Parkinsonism, or dystonia of the central nervous system; arrhythmia and hypocalcemia-related dilated cardiomyopathy of the cardiovascular system; anxiety and depression of the neuropsychiatric system; and cataracts and papilledema of the ophthalmic system. In addition, it includes nephrocalcinosis, kidney stones, and chronic kidney disease in the urinary system, as well as calcification of the central nervous system, which are caused by the long-term use of large doses of active vitamin D and/or calcium supplements in conventional treatment. In relevant studies, conventional treatments for HP include active vitamin D and calcium supplements, during which patients take large amounts of calcium to increase intestinal calcium absorption and thereby raise serum calcium concentrations. Although conventional treatment with active vitamin D and calcium supplements can restore serum calcium levels, it cannot restore other effects of PTH, such as bone turnover or renal calcium reabsorption. Furthermore, conventional treatment carries a certain risk of hypercalciuria, which increases the risk of nephrocalcinosis and kidney stones, as well as long-term complications (including nephrocalcinosis, kidney stones, and intracranial calcification). Therefore, numerous pharmaceutical companies are actively exploring hormone replacement therapies to achieve better treatment outcomes.

To establish a more physiological replacement therapy compared with traditional treatments, research aimed at replacing parathyroid hormone initially begin with the synthetic human parathyroid hormone PTH(1-34), which is the biologically active amino-terminal fragment of the full-length parathyroid hormone peptide. Both the active amino-terminal fragment and the full-length 84-amino acid peptide bind to and activate the parathyroid hormone 1 receptor (PTH1R). PTH(1-34) is the final active molecular form of parathyroid hormone in the human body, while PTH(1-84) is its precursor form. PTH(1-84) exerts its biological functions after being enzymatically processed into PTH(1-34) in vivo. Currently, drugs in the form of PTH(1-34), such as Teriparatide^{®} (Eli Lilly), and drugs in the form of PTH(1-84), such as NATPARA^{®} (Takeda), have been approved for marketing, with half-lives of 30 minutes and 3 hours in the human body, respectively. Due to their short half-lives, i.e., elevating serum calcium in vivo for only 2 hours, clinical studies are conducted via pump delivery or twice-daily injection regimens. PTH(1-84) has a half-life of 3 hours, and can maintain the serum calcium of patient at normal physiological levels within 12 hours after administration. However, since PTH(1-84) cannot maintain normal serum calcium levels throughout the day, patients still need to take active vitamin D and calcium supplements daily, and may also experience short-term and long-term side effects associated with calcium supplement therapy. In addition, PTH(1-84) is only approved for patients whose conditions cannot be controlled by conventional calcium supplement therapy. Moreover, due to the poor stability of the PTH molecule itself, all currently marketed drugs, including the PTH(1-84) drug NATPARA^{®} (Takeda), the PTH(1-34) drug Teriparatide^{®} (Xinlitai Pharmaceutical Co., Ltd, China) , and the PTH(1-34) drug OstloGen^{®} (United Cell Biotechnology Co., Ltd., China) are supplied as lyophilized powders. Patients need to dissolve these drugs with sterile water for injection before each use, making the administration extremely inconvenient.

Technically, neither of the above two treatment methods can fully meet patients' needs. In particular, short-term and long-term toxic and side effects can cause certain harm to patients and affect their quality of life. Moreover, patients with hypoparathyroidism who require drug intervention are generally suffering from permanent hypoparathyroidism and need lifelong medication. Therefore, scientists and pharmaceutical companies worldwide are conducting various studies on new-generation treatment methods. Among them, the most advanced research involves Transcon PTH(1-34) by Ascendis Pharma A/S (Denmark) and LA-PTH(1-34) by Amolyt Pharma (France). Transcon PTH(1-34) is a polyethylene glycol (PEG)-conjugated prodrug, in which two PEG molecules are linked to PTH(1-34) via a linker to form an inactive prodrug. After administration, the PEG moieties are gradually cleaved in vivo to release the PTH molecules, thereby extending the drug half-life of PTH and maintaining normal physiological serum calcium levels within 24 hours. Internationally, Phase III clinical trials of Transcon PTH(1-34) was completed in 2022 and a New Drug Application (NDA) was submitted to the Food and Drug Administration (FDA). In China, VISEN Pharmaceuticals (Shanghai) Co., Ltd., a subsidiary of Ascendis, completed Phase III clinical trial enrollment in June 2022. LA-PTH(1-34) is a peptide analog of parathyroid hormone (PTH), obtained by fusing mutated PTH(1-14) with PTHrP(15-36), which can target parathyroid hormone receptor 1 (PTHR1) with a specific conformation. Compared with PTH(1-34), LA-PTH has enhanced binding affinity to PTHR1. Therefore, although its drug half-life remains unchanged, LA-PTH can increase serum calcium levels for a longer period, safely and effectively maintaining normal serum calcium levels for 24 hours, thereby controlling hypoparathyroidism. In 2022, Phase II clinical trial of LA-PTH was completed, and currently it is preparing for Phase III clinical trials.

Despite the improvements in the efficacy of hypoparathyroidism-related therapeutic drugs, their preparation remains extremely challenging due to the inherent characteristics of PTH. For example, as a PEG-conjugated prodrug, Transcon PTH(1-34) benefits from PEGylation in enhancing solubility and stability. However, Transcon PTH(1-34) requires solid-phase peptide synthesis followed by PEGylation modification, resulting in extremely high costs for both peptide synthesis and conjugation. Similarly, LA-PTH is produced via chemical solid-phase synthesis, leading to high production costs. Additionally, due to the extremely low solubility of PTHrP, primarily attributed to the PTHrP(15-36) segment, which is the binding region for the extracellular domain (ECD) of the PTHR1 receptor, Amolyt Pharma was forced to introduce mutations to improve its solubility, potentially compromising its stability.

Based on the above, there is an urgent need to develop a therapeutic drug for hypoparathyroidism that can extend the duration of pharmacological action, reduce dosage, and exhibit excellent stability and solubility.

### SUMMARY

The present disclosure aims to solve at least one of the technical problems existing in the existing technologies. For this purpose, the present disclosure provides a recombinant PTH fusion protein, which can effectively enhance the duration and potency of pharmacological action, and has excellent temperature stability and freeze-thaw stability, and can be used to treat and/or prevent hypoparathyroidism.

The present disclosure further provides a method for constructing a recombinant PTH fusion protein.

The present disclosure further provides a biomaterial.

The present disclosure further provides use of the recombinant PTH fusion protein in preparing a product for the treatment and/or prevention of hypoparathyroidism.

The present disclosure further provides a drug.

The present disclosure further provides a combination drug.

In a first aspect, the present disclosure provides a recombinant PTH fusion protein including:
A1) at least one signaling domain molecule capable of activating a PTHR1 intracellular pathway;
A2) one or more molecules capable of binding to an extracellular domain (ECD) of PTHR1; and
A3) a human serum albumin nanobody;
wherein an amino acid sequence of the human serum albumin nanobody is shown as SEQ ID NO: 7.

In accordance with the embodiments of the present disclosure, the recombinant PTH fusion protein has at least the following beneficial effects:
(1) The recombinant PTH fusion protein has high safety and long duration of pharmacodynamic action. Compared with teriparatide (rhPTH(1-34)), the recombinant PTH fusion protein of the present disclosure has higher safety and longer duration of pharmacodynamic action. The serum calcium levels is increased within 2 hours and then restored to normal within 6 hours after injection of teriparatide (rhPTH(1-34)), whereas the serum calcium levels is elevated within 24-48 hours and then restored to normal within 48-72 hours after injection of the recombinant PTH fusion protein of the present disclosure at the same dose. The recombinant PTH fusion protein greatly extends the half-life of PTH drugs, which helps to safely and effectively maintain serum calcium levels, thereby controlling hypoparathyroidism.
(2) The recombinant PTH fusion protein has good temperature stability and can be developed into a liquid dosage form. The recombinant PTH fusion protein of the present disclosure can maintain high stability at 4°C-37°C. In particular, when the fusion protein contains a nanobody of PTHR1 (e.g., VHH22A3) or three extracellular domain molecules, it exhibit better temperature stability and can maintain favorable stability at 4°C-60°C. The recombinant PTH fusion protein can overcome the inconvenience of using freeze-dried powder formulations of currently marketed PTH(1-34) and PTH(1-84) drugs, and improve patient compliance.
(3) The recombinant PTH fusion protein has excellent freeze-thaw stability. The recombinant PTH fusion protein of the present disclosure still retains excellent biological activity after undergoing 5 cycles of freezing and thawing at -80°C and room temperature.

It should be understood that ECD means Extracellular Domain, which refers to the extracellular portion of transmembrane proteins (i.e., the N-terminal extracellular region of membrane proteins).

In some embodiments of the present disclosure, an amino acid sequence of the signaling domain molecule capable of activating the PTHR1 intracellular pathway includes a sequence shown as SEQ ID NO: 1 (i.e., PTH(1-14)).

In some preferred embodiments of the present disclosure, the signaling activation domain molecule capable of activating the PTHR1 intracellular pathway is at least one of PTH(1-14) to PTH(1-34).

In some embodiments of the present disclosure, the PTH(1-14) to PTH(1-34) include: PTH(1-14), PTH(1-15), PTH(1-16), PTH(1-17), PTH(1-18), PTH(1-19), PTH(1-20), PTH(1-21), PTH(1-22), PTH(1-23), PTH(1-24), PTH(1-25), PTH(1-26), PTH(1-27), PTH(1-28), PTH(1-29), PTH(1-30), PTH(1-31), PTH(1-32), PTH(1-33) and PTH(1-34).

In some preferred embodiments of the present disclosure, the signaling activation domain molecule capable of activating the PTHR1 intracellular pathway is PTH(1-14).

In some embodiments of the present disclosure, an amino acid sequence of the molecule capable of binding to the ECD of PTHR1 includes at least one of a sequences shown as SEQ ID NO: 3, SEQ ID NO: 5 or SEQ ID NO: 6.

It should be understood that the sequence shown in SEQ ID NO: 3 is PTH(15-34), the sequence shown in SEQ ID NO: 5 is PTHrP(15-36); and the sequence shown in SEQ ID NO: 6 is VHH22A3, a nanobody of PTHR1 (derived from the patent EP2557090A2).

In some embodiments of the present disclosure, the molecule capable of binding to the ECD of PTHR1 includes at least one of PTH(15-34), PTH(14-34), PTH(13-34), PTH(12-34), PTH(11-34), PTH(10-34), PTH(9-34), PTH(8-34), PTH(7-34), PTH(6-34), PTH(5-34), PTH(4-34), PTH(2-34), PTH(15-84), PTH(14-84), PTH(13-84), PTH(12-84), PTH(11-84), PTH(10-84), PTH(9-84), PTH(8-84), PTH(7-84), PTH(6-84), PTH(5-84), PTH(4-84), PTH(3-84), PTH(2-84), PTHrP(15-36), PTHrP(14-36), PTHrP(13-36), PTHrP(12-36), PTHrP(11-36), PTHrP(10-36), PTHrP(9-36), PTHrP(8-36), PTHrP(7-36), PTHrP(6-36), PTHrP(5-36), PTHrP(4-36), PTHrP(3-36), PTHrP(2-36) and a nanobody of PTHR1.

In some embodiments of the present disclosure, the nanobody of PTHR1 includes VHH22A3, and an amino acid sequence of VHH22A3 is shown as SEQ ID NO: 6 (derived from the patent EP2557090A2).

In some embodiments of the present disclosure, the molecule capable of binding to the ECD of PTHR1 has a different binding site from that of the signaling domain molecule capable of activating the PTHR1 intracellular pathway.

In some embodiments of the present disclosure, the signaling domain molecule capable of activating the PTHR1 intracellular pathway, the molecule capable of binding to the ECD of PTHR1 or the human serum albumin nanobody of the recombinant PTH fusion protein are linked via a linker.

In some embodiments of the present disclosure, the linker comprises an amino acid sequence as shown in any one of SEQ ID NO: 8 to SEQ ID NO:10.

In some embodiments of the present disclosure, the recombinant PTH fusion protein further includes a His tag.

In some embodiments of the present disclosure, the recombinant PTH fusion protein has a nucleotide sequence selected from...

In some embodiments of the present disclosure, the recombinant PTH fusion protein has an amino acid sequence selected from any one of the sequences shown in SEQ ID NO: 12, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29 and SEQ ID NO: 31.

In a second aspect, the present disclosure provides a method for constructing the recombinant PTH fusion protein described in the first aspect, specifically including introducing an expression vector containing an encoding gene of the recombinant PTH fusion protein described in any one of the first aspect into a host cell to express the encoding gene, and performing separation and purification to obtain the recombinant PTH fusion protein.

In some embodiments of the present disclosure, the expression vector includes a pET 30a(+) vector.

In some embodiments of the present disclosure, the host cell includes Escherichia coli.

In some preferred embodiments of the present disclosure, the Escherichia coli is Escherichia coli BL21(DE3).

In a third aspect, the present disclosure provides a biomaterial, and the biomaterial is any one of B1) to B4):
B1) a nucleic acid molecule encoding the recombinant PTH fusion protein according to any one of the first aspect;
B2) an expression cassette containing the nucleic acid molecule described in B1);
B3) a recombinant vector containing the nucleic acid molecule described in B1) or the expression cassette described in B2); and
B4) a host cell containing the nucleic acid molecule described in B1), the expression cassette described in B2), or the recombinant vector described in B3).

In a fourth aspect, the present disclosure provides use of the recombinant PTH fusion protein described in any one of the first aspect in preparing a product for the treatment and/or prevention of hypoparathyroidism.

In a fifth aspect, the present disclosure provides a drug including the recombinant PTH fusion protein described in any one of the first aspect and a pharmaceutically acceptable excipient.

In a sixth aspect, the present disclosure provides a combination drug including at least one of the recombinant PTH fusion protein described in any one of the first aspect and the drug described in the fifth aspect, and an anti-tumor targeting drug.

In a seventh aspect, the present disclosure provides a method for treating and/or preventing hypoparathyroidism, including the following steps:
administering a therapeutically and/or prophylactically effective amount of at least one of the aforementioned recombinant PTH fusion protein, the biomaterial, the drug or the combination drug to a subject in need thereof.

Additional features and advantages of the present disclosure will be shown in the description which follows, and in part will be obvious from the description, or may be learned by the practice of the present disclosure.

### BRIEF DESCRIPTION OF DRAWINGS

The present disclosure will be further described below with reference to the accompanying drawings and embodiments.
Figure 1 is a schematic diagram of a fusion protein of human serum albumin nanobody and PTH protein according to the present disclosure.
Figure 2 is a schematic diagram of a binding mode between the PTH protein and a receptor PTHR1 according to the present disclosure.
Figure 3 is a schematic diagram of the binding of functional molecules with monovalent and multivalent receptor ECD binding domains to the human serum albumin nanobody according to the present disclosure.
Figure 4 is a schematic diagram of the binding and dissociation of the fusion protein with the PTHR1 receptor according to the present disclosure.
Figure 5A and Figure 5B show the results of the animal pharmacodynamic assay of the blank control group according to the present disclosure, wherein Figure 5A shows the results of the time period of 0 h-6 h, and Figure 5B shows the results of the time period of 0 h-96 h.
Figure 6A and Figure 6B show the results of the animal pharmacodynamic assay of the fusion protein T002G-A according to the present disclosure, wherein Figure 6A shows the results of the time period of 0 h-6 h, and Figure 6B shows the results of the time period of 0 h 96 h.
Figure 7A to Figure 7D show the results of animal pharmacodynamic assay of the fusion proteins T002G-B to T002G-I according to the present disclosure, wherein Figure 7A shows the results of fusion proteins T002G-B and T002G-C, Figure 7B shows the results of fusion proteins T002G-D and T002G-E, Figure 7C shows the results of fusion proteins T002G-F and T002G-G, and Figure 7D shows the results of fusion proteins T002G-H and T002G-I.
Figure 8 shows the results of animal pharmacodynamic assay of the fusion proteins T002G-J and T002G-K according to the present disclosure.
Figure 9A and Figure 9B show the results of temperature stability assay of the fusion proteins T002G-A to T002G-K according to the present disclosure, wherein Figure 9A shows the results of fusion proteins T002G-A to T002G-I, and Figure 9B shows the results of fusion proteins T002G-J, T002G-K and NbHSA.
Figure 10A and Figure 10B show the results of freeze-thaw stability assay of the fusion proteins T002G-A to T002G-K according to the present disclosure, wherein Figure10A shows the results of fusion proteins T002G-A to T002G-I, and Figure 10B shows the results of fusion proteins T002G-J, T002G-K and NbHSA.

### DETAILED DESCRIPTION

The concept and technical effects of the present disclosure will be clearly and completely described below in conjunction with the embodiments, so as to fully understand the purpose, features and effects of the present disclosure. Obviously, the described embodiments are only some rather than all embodiments of the present disclosure. All other examples obtained by those skilled in the art without creative efforts based on the embodiments of the present disclosure shall fall within the protection scope of the present disclosure.

When a numerical range is disclosed herein, the aforementioned range shall be regarded as continuous and shall include the minimum and maximum values of the range, as well as every value between such minimum and maximum values. Further, when the range refers to integers, it shall include every integer between the minimum and maximum values of the range. In addition, when multiple ranges are provided to describe a feature or characteristic, the ranges may be combined. In other words, unless otherwise specified, all ranges disclosed herein shall be construed as including any and all subranges subsumed therein.

In the description of the present disclosure, references to the terms "one embodiment", "some embodiments", "exemplary embodiment", "example", "specific example", or "some examples" etc. mean that a specific feature, structure, material or characteristic described in connection with the embodiment or example is included in at least one embodiment or example of the present disclosure. In this specification, the schematic representations of the above terms do not necessarily refer to the same embodiment or example.

The term "nucleotide" generally refers to a compound formed by linking a nucleoside to an acidic molecule or group via an ester bond. For example, phosphate esters of nucleosides usually have one, two or three phosphate groups covalently linked to the 5-position of the sugar group of the nucleoside. In some cases, the definition of nucleotide also includes homologs or analogs of typical nucleotides.

The term "amino acid" refers to the basic unit that constitutes a protein, which endows the protein with specific molecular structural forms and biochemical activity. For example, the "amino acids" used in the present disclosure include the following 20 natural amino acids: alanine (Ala or A), glycine (Gly or G), isoleucine (Ile or I), asparagine (Asn or N), arginine (Arg or R), lysine (Lys or K), cysteine (Cys or C), aspartic acid (Asp or D), glutamic acid (Glu or E), glutamine (Gln or Q), histidine (His or H), leucine (Leu or L), methionine (Met or M), phenylalanine (Phe or F), proline (Pro or P), serine (Ser or S), threonine (Thr or T), tryptophan (Trp or W), valine (Val or V) and tyrosine (Tyr or Y).

Unless otherwise specified, "room temperature" in the present disclosure refers to 25°C±5°C.

In addition, unless specially defined, all scientific or technical terms used in this patent are consistent with the common understanding of most persons skilled in the art.

In the embodiments, the specific conditions not indicated shall be carried out in accordance with conventional conditions or the conditions suggested by the manufacturer. The reagents or instruments not indicated by the manufacturer are all conventional products that can be available commercially.

### Invention Concept

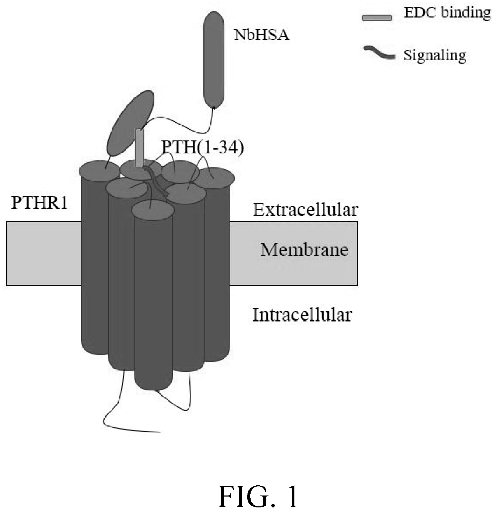

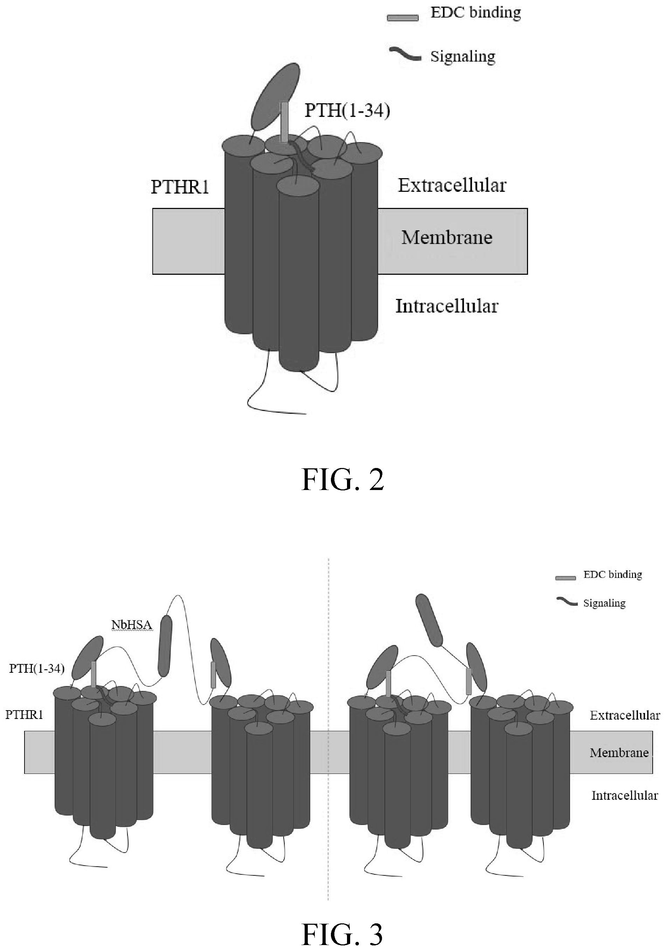

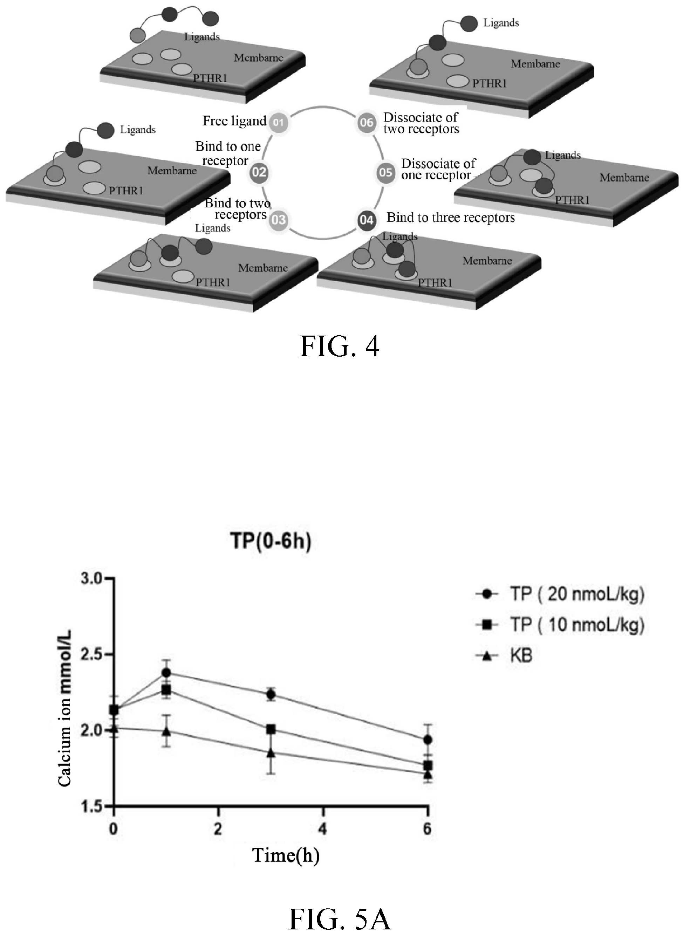

The present disclosure involves discovering that fusing the human serum albumin nanobody NbHSA with the PTH(1-34) molecule (as shown in Figure 1) helps to improve the druggability of PTH(1-34) and prolong its therapeutic effect. Additionally, the high stability of the nanobody improves the stability of PTH(1-34), which facilitates to prepare liquid dosage form drugs, improves the inconvenience in the use of the marketed lyophilized powder dosage forms of PTH(1-34) and PTH(1-84) molecules, and enhances patient compliance. Furthermore, through the analysis of the binding mode between the PTH(1-34) molecule and the receptor PTHR1 (as shown in Figure 2), the present disclosure finds that the PTH(1-34) interacts with the receptor in a domain-specific interaction mode, specifically, PTH(15-34) first binds to the extracellular domain of PTHR1, and then the signaling domain molecule PTH(1-14) capable of activating the PTHR1 intracellular pathway activates the receptor. The present disclosure creatively designs functional molecules with monovalent and multivalent receptor ECD-binding domain modes to fuse with human serum albumin nanobody (as shown in Figure 3). On the basis of ensuring the drug half-life, by enhancing the affinity between the drug molecule and the receptor, the pharmacological action duration is prolonged (as shown in Figure 4), the drug dosage is further reduced, and the duration of elevated drug molecule concentration is prolonged.

The recombinant PTH fusion protein molecule of the present disclosure is designed into structures similar to A-C, A-B-C, A-C-B, A-B-C-B, etc., wherein:
A is a signaling domain molecule capable of activating PTHR1, including sequence molecules ranging from PTH(1-14) to PTH(1-34);
B is molecules capable of binding to the ECD of PTHR1, including but not limited to PTH(15-34), PTH(14-36), PTHrP(1-36), PTHrP(15-36) and nanobodies of PTHR1 (e.g., VHH22A3), which can be one or more identical or different binding molecules;
C is a nanobody molecule that binds to human serum albumin, for example, human serum albumin nanobody NbHSA.

The amino acid sequence of PTH(1-14) is:
SVSEIQLMHNLGKH (SEQ ID NO: 1);

The amino acid sequence of PTH(1-34) is:
SVSEIQLMHNLGKHLNSMERVEWLRKKLQDVHNF (SEQ ID NO: 2);

The amino acid sequence of PTH(15-34) is:
LNSMERVEWLRKKLQDVHNF (SEQ ID NO: 3);

The amino acid sequence of PTHrP(1-36) is:
AVSEHQLLHDKGKSIQDLRRRFFLHHLIAEIHTAEI (SEQ ID NO: 4);

The amino acid sequence of PTHrP(15-36) is:
IQDLRRRFFLHHLIAEIHTAEI (SEQ ID NO: 5);

The amino acid sequence of VHH22A3, the nanobody of PTHR1, is:

EVQLVESGGGLVQAGGSLRLSCAASGNIFANNIMGWYRQPPGKEREFVAHVSHDGDS
MYAVSVKGRFAISRKDATNLYLQMNSLKPEDTAIYFCRLLNIPTQGRMEGFWGQGTQVTV
SS (SEQ ID NO: 6);

The amino acid sequence of the human serum albumin nanobody NbHSA is:

QVQLVESGGGLVQTGGSLRLSCAASGSTLVGHSVGFFRQAPGKEREPVVCISSTETVM
YYTNSVKGRFTISRDNAKNTVWLQMDRLAPEDTAVYYCAARPQLCSALDYRYNSWGPGT
QVTVSS (SEQ ID NO: 7).

The aforementioned A, B and C are linked by a linker, and the linker includes but not limited to: 5' -GGGGS-3', 5'-GGGGSVDDDDK-3' (SEQ ID NO: 8), 5'-GGGGSGGGS-3' (SEQ ID NO: 9), 5'-GGGGSGGGGS-3' (SEQ ID NO: 10) or 5'-GGGGSGGGGSGGGGS-3' (SEQ ID NO: 33).

The inventive concept will be described in detail below in conjunction with specific embodiments.

### Example 1: Fusion Protein of NbHSA and PTH

A mono-ECD-binding fusion protein T002G-A, which contained a NbHSA and a PTH(1-34) protein, was provided in this example. The preparation of the fusion protein T002G-A in this example included the following process.

### 1. Construction of recombinant strain

General Biol (Anhui) Co., Ltd. (China) was commissioned to obtain the encoding nucleotide sequence (with his tag) of mono-ECD-binding fusion protein T002G-A via chemical synthesis. The sequence information was as follows:

5′-ATGGAATTTAAACGTAAACGGCATCACCATCACCATCACGGTGGAGGCGGGAGTGTAGACGA
TGACGATAAGTCCGTCTCCGAGATTCAGCTGATGCACAACCTGGGCAAACACCTGAACTCCATGGAA
CGCGTGGAATGGCTGCGTAAAAAGCTGCAGGATGTTCATAACTTCGGTGGTGGCGGTTCCGGTGGCG
GTTCTCAGGTGCAGCTGGTGGAAAGCGGCGGTGGCCTGGTTCAGACCGGCGGTAGTCTGCGCCTGAG
CTGCGCAGCAAGTGGCAGTACCCTGGTGGGCCATAGTGTGGGCTTTTTCCGTCAGGCACCGGGTAAA
GAACGTGAACCGGTTGTTTGCATTAGCAGTACCGAAACCGTTATGTATTATACCAATAGTGTGAAAGG
CCGCTTTACCATTAGTCGCGATAATGCAAAAAATACCGTTTGGCTGCAGATGGATCGCCTGGCCCCGG
AAGATACCGCAGTGTATTATTGTGCCGCACGCCCGCAGCTGTGTAGCGCCTTAGATTATCGTTATAATA
GTTGGGGTCCGGGTACCCAGGTGACCGTTAGTAGT-3′ (SEQ ID NO: 11).

The above fragment was inserted into the prokaryotic expression plasmid pET 30a(+) (Novagen) via *Nde I* and *Xho I* restriction sites, followed by sequencing verification to screen out the recombinant expression plasmid containing the target gene. Then, the recombinant expression plasmid containing the target gene was transformed into *E. coli* BL21 (DE3) competent cells, specifically as follows:
100 µL of BL21 competent cells were thawed on ice, added with the above recombinant expression plasmid containing the target gene, gently mixed, and placed on ice for 30 minutes. Subsequently, heat shock was performed in a 42°C water bath for 30 seconds, after which the centrifuge tube was rapidly transferred to ice bath and placed for 2 minutes without shaking the tube. 400 µL of sterile 2YT medium (containing 16 g/L tryptone, 10 g/L yeast extract, 5 g/L sodium chloride and water, without antibiotics) was added to the centrifuge tube, mixed well, and cultured on a shaker at 37°C and 220 rpm for 1 hour to resuscitate the bacteria. 200 µL of the transformed competent cells were spread onto a 2YT agar medium plate with kanamycin resistance, and the cells were evenly plated. The plate was cultured overnight at 37°C. On the next day, single colonies on the transformation plate were picked using an inoculation loop, and positive clones were screened and inoculated into 15 mL of sterile 2YT medium (containing kanamycin), and cultured overnight at 30°C to obtain the recombinant strain.

### 2. Expression of fusion protein

The above recombinant strain was inoculated onto a 2YT plate containing kanamycin using the streak plate method and incubated overnight in a 37°C thermostatic incubator. On the next day, monoclonal strains were picked and placed in 20 mL of 2YT liquid medium containing kanamycin, followed by shaking culture at 37°C until the OD₆₀₀ value of the bacterial solution reached 0.4-0.6. Then, the bacterial solution was inoculated into 500 mL of 2YT liquid medium containing kanamycin at a ratio of 1:100, and cultured with shaking at 37°C until the OD₆₀₀ value of the bacterial solution reached 4-6. Isopropylthio-β-D-galactoside (IPTG) with a final concentration of 0.5 mM was added for induction. The culture was continued overnight at 18°C, and then the bacterial cells were collected to obtain the recombinant strain expressing the fusion protein.

When *E. coli* BL21 (DE3) was used as the host, the expressed fusion protein T002G-A accounted for approximately 30% of the total bacterial protein, and existed mainly in the form of soluble protein.

### 3. Purification and enzymatic digestion of fusion protein

The bacterial cells of the recombinant strain expressing the fusion protein were resuspended in 50 mmol/L Tris-HCl (pH 8.0) at a ratio of 1:30, and then performing disruption three times using a high-pressure homogenizer at 1000 bar. After disruption, the sample was centrifuged at 8000 rpm for 30 min, the insoluble precipitate was discarded, and the supernatant was retained for further purification.

Ni 4FF resin was used for target protein purification. The loading buffer was 20 mmol/L Tris-HCl (pH 8.0) equilibrated with 0.3 M NaCl. After loading, the sample was washed with 20 mmol/L Tris-HCl (pH 8.0), 0.3 M NaCl, and 30 mM imidazole to remove impurities and non-specifically bound proteins. Finally, the target protein T002G-A was eluted with 20 mmol/L Tris-HCl (pH 8.0), 0.3 M NaCl, and 250 mM imidazole.

Enterokinase (EK) was added to the T002G-A fusion protein purified by Ni column affinity chromatography at a mass ratio of 1:1000, followed by enzymatic digestion at 25°C overnight (18 h). After digestion, the sample was diluted 10-fold with 20 mmol/L Tris-HCl (pH 8.0). The diluted sample was subjected to flow-through purification with Ni 4FF resin, where the target molecule T002G-A obtained after enzymatic digestion was collected in the flow-through fraction, while the undigested T002G-A fusion protein and EK were captured by Ni column affinity. The flow-through sample was concentrated using an ultrafiltration concentration tube and buffer-exchanged to obtain the mono-ECD-binding fusion protein T002G-A.

Sequence analysis was performed on the above mono-ECD-binding fusion protein T002G-A, with the amino acid sequence information as follows:

MEFKRKRHHHHHHGGGGSVDDDKSVSEIQLMHNLGKHLNSMERVEWLRKKLQD
VHNFGGGGSGGGSQVQLVESGGGLVQTGGSLRLSCAASGSTLVGHSVGFFRQAPGKEREP
VVCISSTETVMYYTNSVKGRFTISRDNAKNTVWLQMDRLAPEDTAVYYCAARPQLCSALD
YRYNSWGPGTQVTVSS (SEQ ID NO: 12).

The amino acid sequence in bold and underlined represented the linker.

The results were consistent with the expectations, indicating that the mono-ECD-binding fusion protein T002G-A was successfully prepared in this example.

### Example 2: PTH(1-34)-PTH(15-34)-NbHSA

This example provided a dual-ECD-binding fusion protein T002G-B, which was obtained by linking PTH(1-34), PTH(15-34) and anti-HSA nanobody in sequence. Its amino acid sequence information was as follows:

MEFKRKRHHHHHHGGGGSVDDDKSVSEIQLMHNLGKHLNSMERVEWLRKKLQDV
HNFGGGGSGGGSLNSMERVEWLRKKLQDVHNFGGGGSGGGSQVQLVESGGGLVQTGGSL
RLSCAASGSTLVGHSVGFFRQAPGKEREPVVCISSTETVMYYTNSVKGRFTISRDNAKNTV
WLQMDRLAPEDTAVYYCAARPQLCSALDYRYNSWGPGTQVTVSS (SEQ ID NO: 13).

The preparation process of the fusion protein T002G-B in this example was carried out with reference to the preparation method of the mono-ECD-binding fusion protein T002G-A described in Example 1. The encoding nucleotide sequence information of the fusion protein T002G-B was as follows:

5′-ATGGAATTTAAACGTAAACGGCATCACCATCACCATCACGGTGGAGGCGGGAGT
GTAGACGATGACGATAAGTCCGTCTCCGAGATTCAGCTGATGCACAACCTGGGCAAACA
CCTGAACTCCATGGAACGCGTGGAATGGCTGCGTAAAAAGCTGCAGGATGTTCATAACT
TCGGTGGTGGCGGTAGCGGCGGTAGTCTGAATAGCATGGAACGTGTTGAATGGCTG
CGCAAAAAACTGCAAGATGTTCATAATTTTGGTGGTGGCGGTTCCGGTGGCGGTTCTCA
GGTGCAGCTGGTGGAAAGCGGCGGTGGCCTGGTTCAGACCGGCGGTAGTCTGCGCCTG
AGCTGCGCAGCAAGTGGCAGTACCCTGGTGGGCCATAGTGTGGGCTTTTTCCGTCAGGC

ACCGGGTAAAGAACGTGAACCGGTTGTTTGCATTAGCAGTACCGAAACCGTTATGTATTA
TACCAATAGTGTGAAAGGCCGCTTTACCATTAGTCGCGATAATGCAAAAAATACCGTTTG
GCTGCAGATGGATCGCCTGGCCCCGGAAGATACCGCAGTGTATTATTGTGCCGCACGCCC
GCAGCTGTGTAGCGCCTTAGATTATCGTTATAATAGTTGGGGTCCGGGTACCCAGGTGAC
CGTTAGTAGT-3′ (SEQ ID NO: 14).

### Example 3: PTH(1-34)-NbHSA-PTH(15-34)

This example provided a dual-ECD-binding fusion protein T002G-C, which was obtained by linking PTH(1-34), an anti-HSA nanobody, and PTH(15-34) in sequence. Its amino acid sequence information was as follows:

MEFKRKRHHHHHHGGGGSVDDDDKSVSEIQLMHNLGKHLNSMERVEWLRKKLQDV
HNFGGGGSGGGSQVQLVESGGGLVQTGGSLRLSCAASGSTLVGHSVGFFRQAPGKEREPV
VCISSTETVMYYTNSVKGRFTISRDNAKNTVWLQMDRLAPEDTAVYYCAARPQLCSALDY
RYNSWGPGTQVTVSSGGGGSGGGSLNSMERVEWLRKKLQDVHNF (SEQ ID NO: 15).

The preparation process of the fusion protein T002G-C in this example was carried out with reference to the preparation method of the mono-ECD-binding fusion protein T002G-A described in Example 1. The encoding nucleotide sequence information of the fusion protein T002G-C was as follows:

5′-ATGGAATTTAAACGTAAACGGCATCACCATCACCATCACGGTGGAGGCGGGAGT
GTAGACGATGACGATAAGTCCGTCTCCGAGATTCAGCTGATGCACAACCTGGGCAAACA
CCTGAACTCCATGGAACGCGTGGAATGGCTGCGTAAAAAGCTGCAGGATGTTCATAACT
TCGGTGGTGGCGGTTCCGGTGGCGGTTCTCAGGTGCAGCTGGTGGAAAGCGGCGGTGG
CCTGGTTCAGACCGGCGGTAGTCTGCGCCTGAGCTGCGCAGCAAGTGGCAGTACCCTG
GTGGGCCATAGTGTGGGCTTTTTCCGTCAGGCACCGGGTAAAGAACGTGAACCGGTTGT
TTGCATTAGCAGTACCGAAACCGTTATGTATTATACCAATAGTGTGAAAGGCCGCTTTACC
ATTAGTCGCGATAATGCAAAAAATACCGTTTGGCTGCAGATGGATCGCCTGGCCCCGGA
AGATACCGCAGTGTATTATTGTGCCGCACGCCCGCAGCTGTGTAGCGCCTTAGATTATCG
TTATAATAGTTGGGGTCCGGGTACCCAGGTGACCGTTAGTAGTGGTGGTGGCGGTAGCG
GCGGCGGTAGTCTGAATAGCATGGAACGTGTTGAATGGCTGCGCAAAAAACTGCAAGAT
GTTCATAATTTT-3′ (SEQ ID NO: 16).

### Example 4: PTH(1-34)-PTrP(15-36)-NbHSA

This example provided a dual-ECD-binding fusion protein T002G-D, which was obtained by linking PTH (1-34), PTrP (15-36) and an anti-HSA nanobody in sequence, wherein the linker was SEQ ID NO:33. Its amino acid sequence information was as follows:

MEFKRKRHHHHHHGGGGSVDDDDKSVSEIQLMHNLGKHLNSMERVEWLRKKLQDV

HNFGGGGSGGGSIQDLRRRFFLHHLIAEIHTAEIGGGGSGGGSQVQLVESGGGLVQTGGSLR
LSCAASGSTLVGHSVGFFRQAPGKEREPVVCISSTETVMYYTNSVKGRFTISRDNAKNTVW
LQMDRLAPEDTAVYYCAARPQLCSALDYRYNSWGPGTQVTVSS (SEQ ID NO: 17).

The preparation process of the fusion protein T002G-D in this example was carried out with reference to the preparation method of the mono-ECD-binding fusion protein T002G-A described in Example 1. The encoding nucleotide sequence information of the fusion protein T002G-D was as follows:

5′-ATGGAATTTAAACGTAAACGGCATCACCATCACCATCACGGTGGAGGCGGGAGT
GTAGACGATGACGATAAGTCCGTCTCCGAGATTCAGCTGATGCACAACCTGGGCAAACA
CCTGAACTCCATGGAACGCGTGGAATGGCTGCGTAAAAAGCTGCAGGATGTTCATAACT
TCGGTGGTGGCGGTAGCGGCGGTAGTATTCAGGATCTGCGCCGTCGCTTTTTCCTGC
ATCATCTGATTGCAGAAATTCATACCGCCGAAATTGGTGGTGGCGGTTCCGGTGGCGGTT
CTCAGGTGCAGCTGGTGGAAAGCGGCGGTGGCCTGGTTCAGACCGGCGGTAGTCTGCG
CCTGAGCTGCGCAGCAAGTGGCAGTACCCTGGTGGGCCATAGTGTGGGCTTTTTCCGTC
AGGCACCGGGTAAAGAACGTGAACCGGTTGTTTGCATTAGCAGTACCGAAACCGTTATG
TATTATACCAATAGTGTGAAAGGCCGCTTTACCATTAGTCGCGATAATGCAAAAAATACC
GTTTGGCTGCAGATGGATCGCCTGGCCCCGGAAGATACCGCAGTGTATTATTGTGCCGCA
CGCCCGCAGCTGTGTAGCGCCTTAGATTATCGTTATAATAGTTGGGGTCCGGGTACCCAG
GTGACCGTTAGTAGT-3′ (SEQ ID NO: 18).

### Example 5: PTH(1-34)-NbHSA-PTHrP(15-36)

This example provided a dual-ECD-binding fusion protein T002G-E, which was obtained by linking PTH (1-34), an anti-HSA nanobody and PTrP (15-36) in sequence. Its amino acid sequence information was as follows:

MEFKRKRHHHHHHGGGGSVDDDDKSVSEIQLMHNLGKHLNSMERVEWLRKKLQDV
HNFGGGGSGGGSQVQLVESGGGLVQTGGSLRLSCAASGSTLVGHSVGFFRQAPGKEREPV
VCISSTETVMYYTNSVKGRFTISRDNAKNTVWLQMDRLAPEDTAVYYCAARPQLCSALDY
RYNSWGPGTQVTVSSGGGGSGGGSIQDLRRRFFLHHLIAEIHTAEI (SEQ ID NO: 19).

The preparation process of the fusion protein T002G-E in this example was carried out with reference to the preparation method of the mono-ECD-binding fusion protein T002G-A described in Example 1. The encoding nucleotide sequence information of the fusion protein T002G-E was as follows:

5′-ATGGAATTTAAACGTAAACGGCATCACCATCACCATCACGGTGGAGGCGGGAGT
GTAGACGATGACGATAAGTCCGTCTCCGAGATTCAGCTGATGCACAACCTGGGCAAACA
CCTGAGGTGGCGGTTCCGGTGGCGGTTCTCAGGTGCAGCTGGTGGAAAGCGGCGGTGG

CCTGGTTCAGACCGGCGGTAGTCTGCGCCTGAGCTGCGCAGCAAGTGGCAGTACCCTG
GTGGGCCATAGTGTGGGCTTTTTCCGTCAGGCACCGGGTAAAGAACGTGAACCGGTTGT
TTGCATTAGCAGTACCGAAACCGTTATGTATTATACCAATAGTGTGAAAGGCCGCTTTACC
ATTAGTCGCGATAATGCAAAAAATACCGTTTGGCTGCAGATGGATCGCCTGGCCCCGGA
AGATACCGCAGTGTATTATTGTGCCGCACGCCCGCAGCTGTGTAGCGCCTTAGATTATCG
TTATAATAGTTGGGGTCCGGGTACCCAGGTGACCGTTAGTAGTGGTGGTGGCGGTAGCG
GCGGCGGTAGTATTCAGGATCTGCGCCGTCGCTTTTTCCTGCATCATCTGATTGCAGAAA
TTCATACCGCCGAAATT-3′ (SEQ ID NO: 20).

### Example 6: PTH(1-34)-VHH22A3-NbHSA

This example provided a dual-ECD-binding fusion protein T002G-F, which was obtained by linking PTH(1-34), VHH22A3, and an anti-HSA nanobody in sequence, wherein VHH22A3 was an anti-PTHR1 nanobody. The amino acid sequence information of the fusion protein T002G-F was as follows:

MEFKRKRHHHHHHGGGGSVDDDDKSVSEIQLMHNLGKHLNSMERVEWLRKKLQDV
HNFGGGGSGGGSEVQLVESGGGLVQAGGSLRLSCAASGNIFANNIMGWYRQPPGKEREFV
AHVSHDGDSMYAVSVKGRFAISRKDATNLYLQMNSLKPEDTAIYFCRLLNIPTQGRMEGFW
GQGTQVTVSSGGGGSGGGSQVQLVESGGGLVQTGGSLRLSCAASGSTLVGHSVGFFRQAP
GKEREPVVCISSTETVMYYTNSVKGRFTISRDNAKNTVWLQMDRLAPEDTAVYYCAARPQ
LCSALDYRYNSWGPGTQVTVSS (SEQ ID NO: 21).

The preparation process of the fusion protein T002G-F in this example was carried out with reference to the preparation method of the mono-ECD-binding fusion protein T002G-A described in Example 1. The encoding nucleotide sequence information of the fusion protein T002G-F was as follows:

5′-ATGGAATTTAAACGTAAACGGCATCACCATCACCATCACGGTGGAGGCGGGAGT
GTAGACGATGACGATAAGTCCGTCTCCGAGATTCAGCTGATGCACAACCTGGGCAAACA
CCTGAACTCCATGGAACGCGTGGAATGGCTGCGTAAAAAGCTGCAGGATGTTCATAACT
TCGGTGGTGGCGGTAGCGGCGGTAGTGAAGTTCAGCTGGTTGAAAGCGGTGGTGG
CCTGGTTCAGGCAGGTGGCAGTCTGCGCCTGAGTTGTGCCGCAAGCGGTAATATTTTTG
CAAATAATATCATGGGCTGGTATCGCCAGCCGCCGGGTAAAGAACGCGAATTTGTGGCC
CATGTTAGCCATGATGGTGACAGCATGTATGCAGTTAGTGTTAAAGGTCGCTTTGCAATT
AGCCGTAAAGATGCCACCAATCTGTATCTGCAGATGAATAGCCTGAAACCGGAAGATAC
CGCAATCTATTTTTGTCGTCTGCTGAATATTCCGACCCAGGGCCGTATGGAAGGTTTTTG
GGGTCAGGGTACCCAGGTGACCGTTAGTAGTGGTGGTGGCGGTTCCGGTGGCGGTTCTC
AGGTGCAGCTGGTGGAAAGCGGCGGTGGCCTGGTTCAGACCGGCGGTAGTCTGCGCCT

GAGCTGCGCAGCAAGTGGCAGTACCCTGGTGGGCCATAGTGTGGGCTTTTTCCGTCAGG
CACCGGGTAAAGAACGTGAACCGGTTGTTTGCATTAGCAGTACCGAAACCGTTATGTATT
ATACCAATAGTGTGAAAGGCCGCTTTACCATTAGTCGCGATAATGCAAAAAATACCGTTT
GGCTGCAGATGGATCGCCTGGCCCCGGAAGATACCGCAGTGTATTATTGTGCCGCACGC
CCGCAGCTGTGTAGCGCCTTAGATTATCGTTATAATAGTTGGGGTCCGGGTACCCAGGTG
ACCGTTAGTAGT-3′ (SEQ ID NO: 22).

### Example 7: PTH(1-34)-NbHSA-VHH22A3

This example provided a dual-ECD-binding fusion protein T002G-G, which was obtained by linking PTH(1-34), an anti-HSA nanobody and VHH22A3 in sequence, wherein VHH22A3 was an anti-PTHR1 nanobody. The amino acid sequence information of the fusion protein T002G-G was as follows:

MEFKRKRHHHHHHGGGGSVDDDDKSVSEIQLMHNLGKHLNSMERVEWLRKKLQDV
HNFGGGGSGGGSQVQLVESGGGLVQTGGSLRLSCAASGSTLVGHSVGFFRQAPGKEREPV
VCISSTETVMYYTNSVKGRFTISRDNAKNTVWLQMDRLAPEDTAVYYCAARPQLCSALDY
RYNSWGPGTQVTVSSGGGGSGGGSEVQLVESGGGLVQAGGSLRLSCAASGNIFANNIMGW
YRQPPGKEREFVAHVSHDGDSMYAVSVKGRFAISRKDATNLYLQMNSLKPEDTAIYFCRLL
NIPTQGRMEGFWGQGTQVTVSS (SEQ ID NO: 23).

The preparation process of the fusion protein T002G-G in this example was carried out with reference to the preparation method of the mono-ECD-binding fusion protein T002G-A described in Example 1. The encoding nucleotide sequence information of the fusion protein T002G-G was as follows:

5′-ATGGAATTTAAACGTAAACGGCATCACCATCACCATCACGGTGGAGGCGGGAGT
GTAGACGATGACGATAAGTCCGTCTCCGAGATTCAGCTGATGCACAACCTGGGCAAACA
CCTGAACTCCATGGAACGCGTGGAATGGCTGCGTAAAAAGCTGCAGGATGTTCATAACT
TCGGTGGTGGCGGTTCCGGTGGCGGTTCTCAGGTGCAGCTGGTGGAAAGCGGCGGTGG
CCTGGTTCAGACCGGCGGTAGTCTGCGCCTGAGCTGCGCAGCAAGTGGCAGTACCCTG
GTGGGCCATAGTGTGGGCTTTTTCCGTCAGGCACCGGGTAAAGAACGTGAACCGGTTGT
TTGCATTAGCAGTACCGAAACCGTTATGTATTATACCAATAGTGTGAAAGGCCGCTTTACC
ATTAGTCGCGATAATGCAAAAAATACCGTTTGGCTGCAGATGGATCGCCTGGCCCCGGA
AGATACCGCAGTGTATTATTGTGCCGCACGCCCGCAGCTGTGTAGCGCCTTAGATTATCG
TTATAATAGTTGGGGTCCGGGTACCCAGGTGACCGTTAGTAGTGGTGGTGGCGGTAGCG
GCGGCGGTAGTGAAGTTCAGCTGGTTGAAAGCGGTGGTGGCCTGGTTCAGGCAGGTGG
CAGTCTGCGCCTGAGTTGTGCCGCAAGCGGTAATATTTTTGCAAATAATATCATGGGCTG
GTATCGCCAGCCGCCGGGTAAAGAACGCGAATTTGTGGCCCATGTTAGCCATGATGGTG

ACAGCATGTATGCAGTTAGTGTTAAAGGTCGCTTTGCAATTAGCCGTAAAGATGCCACCA
ATCTGTATCTGCAGATGAATAGCCTGAAACCGGAAGATACCGCAATCTATTTTTGTCGTCT
GCTGAATATTCCGACCCAGGGCCGTATGGAAGGTTTTTGGGGTCAGGGTACCCAGGTGA
CCGTTAGTAGT-3′ (SEQ ID NO: 24).

### Example 8: PTH(1-34)-PTrP(15-36)-NbHSA

This example provided a dual-ECD-binding fusion protein T002G-H, which was obtained by linking PTH(1-34), PTrP(15-36) and an anti-HSA nanobody in sequence, wherein the linker was 5' -GGGGS-3' . Its amino acid sequence information was as follows:

MEFKRKRHHHHHHGGGGSVDDDDKSVSEIQLMHNLGKHLNSMERVEWLRKKLQDV
HNFGGGGSIQDLRRRFFLHHLIAEIHTAEIGGGGSGGGSQVQLVESGGGLVQTGGSLRLSCA
ASGSTLVGHSVGFFRQAPGKEREPVVCISSTETVMYYTNSVKGRFTISRDNAKNTVWLQM
DRLAPEDTAVYYCAARPQLCSALDYRYNSWGPGTQVTVSS (SEQ ID NO: 25).

The preparation process of the fusion protein T002G-H in this example was carried out with reference to the preparation method of the mono-ECD-binding fusion protein T002G-A described in Example 1. The encoding nucleotide sequence information of the fusion protein T002G-H was as follows:

5′-ATGGAATTTAAACGTAAACGGCATCACCATCACCATCACGGTGGAGGCGGGAGT
GTAGACGATGACGATAAGTCCGTCTCCGAGATTCAGCTGATGCACAACCTGGGCAAACA
CCTGAACTCCATGGAACGCGTGGAATGGCTGCGTAAAAAGCTGCAGGATGTTCATAACT
TCGGTGGTGGCGGTAGCATTCAGGATCTGCGCCGTCGCTTTTTCCTGCATCATCTGATTG
CAGAAATTCATACCGCCGAAATTGGTGGTGGCGGTTCCGGTGGCGGTTCTCAGGTGCAG
CTGGTGGAAAGCGGCGGTGGCCTGGTTCAGACCGGCGGTAGTCTGCGCCTGAGCTGCG
CAGCAAGTGGCAGTACCCTGGTGGGCCATAGTGTGGGCTTTTTCCGTCAGGCACCGGGT
AAAGAACGTGAACCGGTTGTTTGCATTAGCAGTACCGAAACCGTTATGTATTATACCAAT
AGTGTGAAAGGCCGCTTTACCATTAGTCGCGATAATGCAAAAAATACCGTTTGGCTGCA
GATGGATCGCCTGGCCCCGGAAGATACCGCAGTGTATTATTGTGCCGCACGCCCGCAGCT
GTGTAGCGCCTTAGATTATCGTTATAATAGTTGGGGTCCGGGTACCCAGGTGACCGTTAG
TAGT-3′ (SEQ ID NO: 26).

### Example 9: PTH(1-34)-PTrP(15-36)-NbHSA

This example provided a dual-ECD-binding fusion protein T002G-I, which was obtained by linking PTH(1-34), PTrP(15-36) and an anti-HSA nanobody in sequence, wherein the linker was SEQ ID NO:33. Its amino acid sequence information was as follows:

MEFKRKRHHHHHHGGGSVDDDDKSVSEIQLMHNLGKHLNSMERVEWLRKKLQDV
HNFGGGGSGGGGSGGGGSIQDLRRRFFLHHLIAEIHTAEIGGGGSGGGSQVQLVESGGGLV

QTGGSLRLSCAASGSTLVGHSVGFFRQAPGKEREPVVCISSTETVMYYTNSVKGRFTISRDN
AKNTVWLQMDRLAPEDTAVYYCAARPQLCSALDYRYNSWGPGTQVTVSS (SEQ ID NO:
27).

The preparation process of the fusion protein T002G-I in this example was carried out with reference to the preparation method of the mono-ECD-binding fusion protein T002G-A described in Example 1. The encoding nucleotide sequence information of the fusion protein T002G-I was as follows:

5′-ATGGAATTTAAACGTAAACGGCATCACCATCACCATCACGGTGGAGGCGGGAGT
GTAGACGATGACGATAAGTCCGTCTCCGAGATTCAGCTGATGCACAACCTGGGCAAACA
CCTGAACTCCATGGAACGCGTGGAATGGCTGCGTAAAAAGCTGCAGGATGTTCATAACT
TCGGCGGTGGTGGCAGTGGTGGCGGTGGCAGTGGCGGTGGTGGTAGTATTCAGGATCTG
CGCCGTCGCTTTTTCCTGCATCATCTGATTGCAGAAATTCATACCGCCGAAATTGGTGGT
GGCGGTTCCGGTGGCGGTTCTCAGGTGCAGCTGGTGGAAAGCGGCGGTGGCCTGGTTC
AGACCGGCGGTAGTCTGCGCCTGAGCTGCGCAGCAAGTGGCAGTACCCTGGTGGGCCA
TAGTGTGGGCTTTTTCCGTCAGGCACCGGGTAAAGAACGTGAACCGGTTGTTTGCATTA
GCAGTACCGAAACCGTTATGTATTATACCAATAGTGTGAAAGGCCGCTTTACCATTAGTC
GCGATAATGCAAAAAATACCGTTTGGCTGCAGATGGATCGCCTGGCCCCGGAAGATACC
GCAGTGTATTATTGTGCCGCACGCCCGCAGCTGTGTAGCGCCTTAGATTATCGTTATAATA
GTTGGGGTCCGGGTACCCAGGTGACCGTTAGTAGT-3′ (SEQ ID NO: 28).

### Example 10: PTH(1-34)-NbHSA-PTHrP(15-36)-VHH22A3

This example provided a triple-ECD-binding fusion protein T002G-J, which was obtained by linking PTH (1-34), an anti-HSA nanobody, PTrP (15-36), and VHH22A3 molecules in sequence. Its amino acid sequence information was as follows:

MEFKRKRHHHHHHGGGGSVDDDDKSVSEIQLMHNLGKHLNSMERVEWLRKKLQDV
HNFGGGGSGGGSEVQLVESGGGLVQAGGSLRLSCAASGNIFANNIMGWYRQPPGKEREFV
AHVSHDGDSMYAVSVKGRFAISRKDATNLYLQMNSLKPEDTAIYFCRLLNIPTQGRMEGFW
GQGTQVTVSSGGGGSGGGSQVQLVESGGGLVQTGGSLRLSCAASGSTLVGHSVGFFRQAP
GKEREPVVCISSTETVMYYTNSVKGRFTISRDNAKNTVWLQMDRLAPEDTAVYYCAARPQ
LCSALDYRYNSWGPGTQVTVSSGGGGSGGGSIQDLRRRFFLHHLIAEIHTAEI (SEQ ID NO:
29).

The preparation process of the fusion protein T002G-J in this example was carried out with reference to the preparation method of the mono-ECD-binding fusion protein T002G-A described in Example 1. The encoding nucleotide sequence information of the fusion protein T002G-J was as follows:

5′-ATGGAATTTAAACGTAAACGGCATCACCATCACCATCACGGTGGAGGCGGGAGT
GTAGACGATGACGATAAGTCCGTCTCCGAGATTCAGCTGATGCACAACCTGGGCAAACA
CCTGAACTCCATGGAACGCGTGGAATGGCTGCGTAAAAAGCTGCAGGATGTTCATAACT
TCGGTGGTGGCGGTAGCGGCGGCGGTAGTGAAGTTCAGCTGGTTGAAAGCGGTGGTGG
CCTGGTTCAGGCAGGTGGCAGTCTGCGCCTGAGTTGTGCCGCAAGCGGTAATATTTTTG
CAAATAATATCATGGGCTGGTATCGCCAGCCGCCGGGTAAAGAACGCGAATTTGTGGCC
CATGTTAGCCATGATGGTGACAGCATGTATGCAGTTAGTGTTAAAGGTCGCTTTGCAATT
AGCCGTAAAGATGCCACCAATCTGTATCTGCAGATGAATAGCCTGAAACCGGAAGATAC
CGCAATCTATTTTTGTCGTCTGCTGAATATTCCGACCCAGGGCCGTATGGAAGGTTTTTG
GGGTCAGGGTACCCAGGTGACCGTTAGTAGTGGTGGTGGCGGTTCCGGTGGCGGTTCTC
AGGTGCAGCTGGTGGAAAGCGGCGGTGGCCTGGTTCAGACCGGCGGTAGTCTGCGCCT
GAGCTGCGCAGCAAGTGGCAGTACCCTGGTGGGCCATAGTGTGGGCTTTTTCCGTCAGG
CACCGGGTAAAGAACGTGAACCGGTTGTTTGCATTAGCAGTACCGAAACCGTTATGTATT
ATACCAATAGTGTGAAAGGCCGCTTTACCATTAGTCGCGATAATGCAAAAAATACCGTTT
GGCTGCAGATGGATCGCCTGGCCCCGGAAGATACCGCAGTGTATTATTGTGCCGCACGC
CCGCAGCTGTGTAGCGCCTTAGATTATCGTTATAATAGTTGGGGTCCGGGTACCCAGGTG
ACCGTTAGTAGTGGTGGTGGCGGTAGCGGCGGCGGTAGTATTCAGGATCTGCGCCGTCG
CTTTTTCCTGCATCATCTGATTGCAGAAATTCATACCGCCGAAATT-3′ (SEQ ID NO: 30).

### Example 11: PTH(1-34)-NbHSA-VHH22A3-PTHrP(15-36)

This example provided a triple-ECD-binding fusion protein T002G-K, which was obtained by linking PTH (1-34), an anti-HSA nanobody, VHH22A3 and PTrP(15-36) molecules in sequence. Its amino acid sequence information was as follows:

MEFKRKRHHHHHHGGGGSVDDDDKSVSEIQLMHNLGKHLNSMERVEWLRKKLQDV
HNFGGGGSGGGSIQDLRRRFFLHHLIAEIHTAEIGGGGSGGGSQVQLVESGGGLVQTGGSLR
LSCAASGSTLVGHSVGFFRQAPGKEREPVVCISSTETVMYYTNSVKGRFTISRDNAKNTVW
LQMDRLAPEDTAVYYCAARPQLCSALDYRYNSWGPGTQVTVSSGGGGSGGGSEVQLVES
GGGLVQAGGSLRLSCAASGNIFANNIMGWYRQPPGKEREFVAHVSHDGDSMYAVSVKGRF
AISRKDATNLYLQMNSLKPEDTAIYFCRLLNIPTQGRMEGFWGQGTQVTVSS (SEQ ID NO:
31).

The preparation process of the fusion protein T002G-K in this example was carried out with reference to the preparation method of the mono-ECD-binding fusion protein T002G-A described in Example 1. The encoding nucleotide sequence information of the fusion protein T002G-K was as follows:

5′-ATGGAATTTAAACGTAAACGGCATCACCATCACCATCACGGTGGAGGCGGAGT

GTAGACGATGACGATAAGTCCGTCTCCGAGATTCAGCTGATGCACAACCTGGGCAAACA
CCTGAACTCCATGGAACGCGTGGAATGGCTGCGTAAAAAGCTGCAGGATGTTCATAACT
TCGGTGGTGGCGGTAGCGGCGGTAGTATTCAGGATCTGCGCCGTCGCTTTTTCCTGC
ATCATCTGATTGCAGAAATTCATACCGCCGAAATTGGTGGTGGCGGTTCCGGTGGCGGTT
CTCAGGTGCAGCTGGTGGAAAGCGGCGGTGGCCTGGTTCAGACCGGCGGTAGTCTGCG
CCTGAGCTGCGCAGCAAGTGGCAGTACCCTGGTGGGCCATAGTGTGGGCTTTTTCCGTC
AGGCACCGGGTAAAGAACGTGAACCGGTTGTTTGCATTAGCAGTACCGAAACCGTTATG
TATTATACCAATAGTGTGAAAGGCCGCTTTACCATTAGTCGCGATAATGCAAAAAATACC
GTTTGGCTGCAGATGGATCGCCTGGCCCCGGAAGATACCGCAGTGTATTATTGTGCCGCA
CGCCCGCAGCTGTGTAGCGCCTTAGATTATCGTTATAATAGTTGGGGTCCGGGTACCCAG
GTGACCGTTAGTAGTGGTGGTGGCGGTAGCGGCGGTAGTGAAGTTCAGCTGGTTG
AAAGCGGTGGTGGCCTGGTTCAGGCAGGTGGCAGTCTGCGCCTGAGTTGTGCCGCAAG
CGGTAATATTTTTGCAAATAATATCATGGGCTGGTATCGCCAGCCGCCGGGTAAAGAACG
CGAATTTGTGGCCCATGTTAGCCATGATGGTGACAGCATGTATGCAGTTAGTGTTAAAGG
TCGCTTTGCAATTAGCCGTAAAGATGCCACCAATCTGTATCTGCAGATGAATAGCCTGAA
ACCGGAAGATACCGCAATCTATTTTTGTCGTCTGCTGAATATTCCGACCCAGGGCCGTAT
GGAAGGTTTTTGGGGTCAGGGTACCCAGGTGACCGTTAGTAGT-3′ (SEQ ID NO: 32).

### Test Example 1: Biological activity verification

The biological activities of the fusion proteins prepared in Examples 1-11 and NbHSA alone were determined according to the biological activity assay method for teriparatide, specifically including the following steps:
pGL4.29 was transfected into UMR-106 cells expressing PTHR1 and subjected to pressure screening. The obtained stable transfected cell line was used for biological activity detection. Specifically, the stable transfected cells were seeded into a white transparent 96-well plate at a density of 2×10⁵ cells per well and cultured at 37°C for 24 hours. Subsequently, the cells were stimulated with serial concentrations of the fusion protein T002A and the control teriparatide (recombinant human parathyroid hormone rhPTH(1-34), hereinafter referred to as TP). Three replicate wells were set up for each sample. After 6 hours of stimulation, 50 µL of fluorescent substrate was added to each well, and the fluorescence intensity was measured using a microplate reader, with the average value calculated.

The assay results are presented in Table 1.

**Table 1: Biological Activity Assay Results**

| Sample Name | EC₅₀ (pm) |
|---|---|
| T002G-A | 43.400 |
| T002G-B | 47.361 |
| T002G-C | 55.789 |
| T002G-D | 55.817 |
| T002G-E | 56.617 |
| T002G-F | 60.681 |
| T002G-G | 67.159 |
| T002G-H | 53.216 |
| T002G-I | 74.005 |
| T002G-J | 71.282 |
| T002G-K | 61.362 |
| TP(PTH(1-34)) | 24.285 |
| NbHSA | 0.000 |

The results shows that the biological activities of the fusion proteins decrease slightly with the increase in the number of ECD binding domains, which may be attributed to minor steric hindrance in the spatial structure caused by the fusion of multiple ECDs. The mono-ECD-binding molecule TP has an EC50 of 24.285 pm, while the EC50 values of the multiple-ECD-binding fusion proteins ranges from 47.361 to 74.005 pm. However, there is no significant difference in EC50 between the fusion proteins with two ECD binding domains and those with three ECD binding domains, and the length of the linker has little effect on the activities of the samples.

### Test Example 2: Pharmacodynamic assay in animals

Eight-week-old male C57 mice were selected for the experiment. The fusion proteins prepared in Examples 1-11 and the control TP were separately diluted with PBS to the concentrations required for the assay. The fusion proteins and TP were administered via intraperitoneal injection at two dosages: 20 nmol/kg and/or 10 nmol/kg, with 3 mice per group. Animals in the blank control group (KB) received an equal volume of normal saline via intraperitoneal injection. Blood samples were collected at 0 h (before injection) and at 1 h, 3 h, 6 h, 24 h, 48 h, and 72 h after injection. The blood samples were allowed to stand at 4°C for 1 hour, and the centrifuged at 4000 rpm for 5 minutes. The serum was harvested after centrifugation. The calcium ion concentration in the mouse serum was quantitatively determined using an Arsenazo III calcium assay kit (Zhongshan Biogentech, China) according to the manufacturer's instructions.

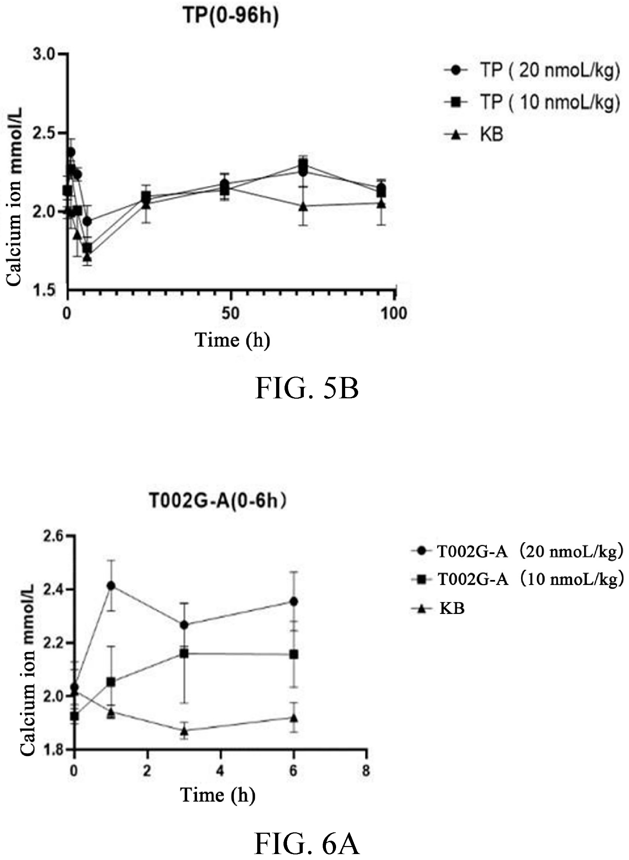

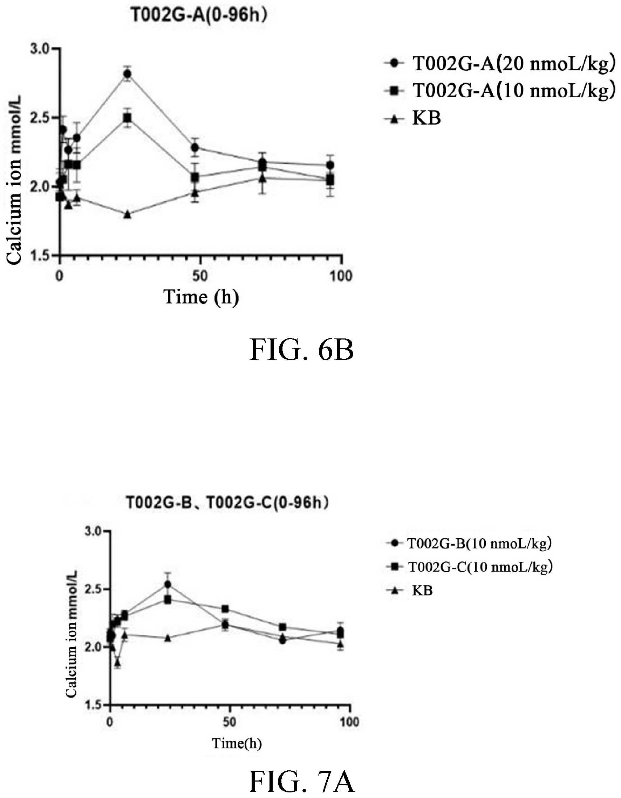

The results of pharmacodynamic assay of TP are shown in Figure 5A and Figure 5B, and the results of pharmacodynamic assay of the mono-ECD-binding fusion protein T002G-A are shown in Figure 6A and Figure 6B. The results indicate that TP at different dosages elevate serum calcium levels within 2 hours and restore to normal within 6 hours; whereas T002G-A at different dosages elevate serum calcium levels within 24 hours and restore to normal within 48 hours. In contrast, the blank control group treated with normal saline exhibits a slight decrease in serum calcium during blood collection, which is probably due to the short interval between successive blood samplings that hinders the recovery of the mice.

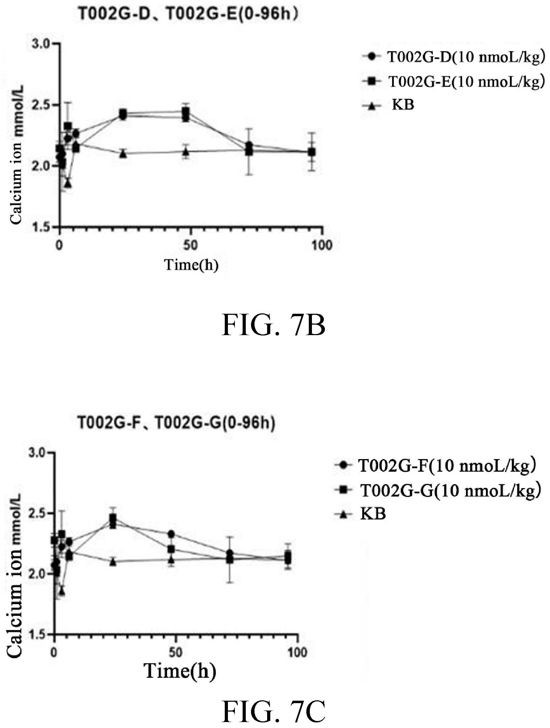

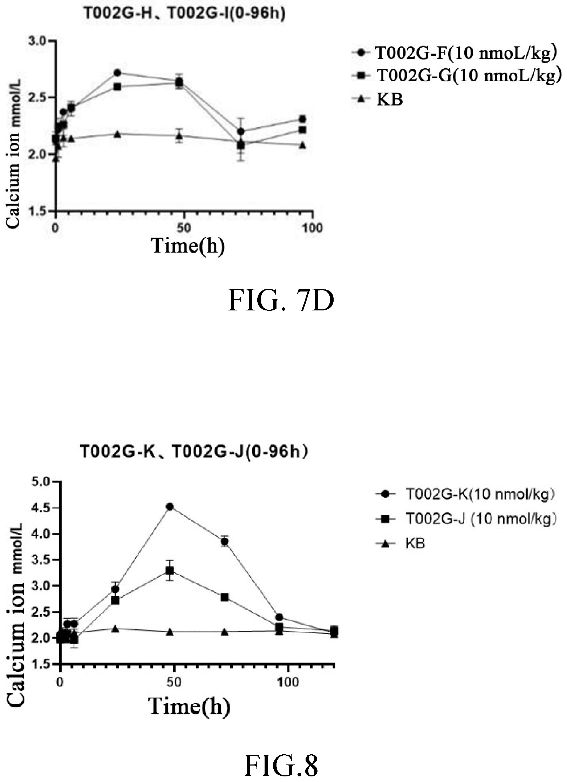

The results of pharmacodynamic assay of the dual-ECD-binding fusion proteins T002G-B to T002G-I are shown in Figure 7A to Figure7D, wherein Figure 7A shows fusion proteins T002G-B and T002G-C, Figure 7B shows fusion proteins T002G-D and T002G-E, Figure 7C shows fusion proteins T002G-F and T002G-G, and Figure 7D shows fusion proteins T002G-H and T002G-I. The results reveal that, compared with the fusion protein T002G-A, further fusion with PTH(15-34) containing the same ECD binding domain does not further enhance the pharmacodynamic duration and intensity (e.g., T002G-B and T002G-C). The calcium-elevating duration remains 24 hours, and the maximum serum calcium concentration is maintained at approximately 2.5 mmol. The reason is possibly that molecules with the same ECD binding domain have identical affinity for receptors on the cell surface; the two ECD-binding molecules bind to and dissociate from the receptors simultaneously, resulting in no extension of the duration for which the signaling domain activates the receptors.

Furthermore, different ECD-binding molecules, including PTHrP(15-36) and VHH22A3, can prolong the pharmacodynamic duration to a certain extent. In particular, PTHrP(15-36), regardless of being fused to the front-end or rear-end of NbHSA (e.g., T002G-D and T002G-E) and regardless of the linker length, extends the calcium-elevating duration to 48 hours, with high serum calcium levels sustained for approximately 72 hours. This may be explained by the differential affinity of different ECD-binding molecules for cell surface receptors: the molecule that first binds to the ECD of the receptor facilitates the binding of the other ECD-binding molecule; after the first bound molecule dissociates, the signaling domain still remains capable of activating the receptor, and the activation only terminates when both differential ECD-binding molecules dissociate. Therefore, the overall duration of receptor activation by the signaling domain is prolonged. However, due to the balance of calcium-elevating and calcium regulation in the mouse body, the maximum serum calcium concentration is still maintained at around 2.5 mmol.

The results of pharmacodynamic assay of the triple-ECD-binding fusion proteins T002G-J and T002G-K are shown in Figure 8. The results reveal that the fusion proteins with three different ECDs has stronger pharmacodynamic action. Especially, T002G-K increase serum calcium levels within 48 hours in mice and maintain high serum calcium levels for 96 hours, with the maximum serum calcium concentration reaching approximately 4.5 mmol.

### Test Example 3: Temperature Stability Study

The temperature stability of the fusion proteins prepared in Examples 1-11 and NbHSA was determined according to the following method:
First, the stock solutions of the fusion proteins prepared in the examples were separately diluted to a concentration of 1 mg/mL (OD concentration) with the corresponding buffer and filtered through a 0.22 µm filter membrane. Each of the diluted solutions was then aliquoted into 8 equal parts, incubated in a water bath at different temperatures: 4°C, 25°C, 37°C, 50°C, 60°C, 70°C, 80°C, and 90°C for 4 hours, placed at room temperature for 2 hours, and then determined for concentration by measuring the absorbance at 280 nm (OD280nm). Finally, the protein concentration of the stock solution under each temperature condition was detected to evaluate the temperature stability, with three replicates set for each group and the average value calculated.

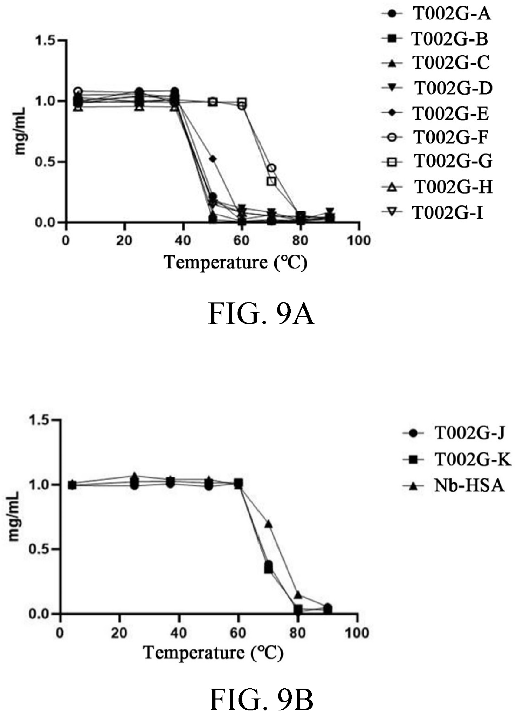

The results are shown in Table 2, Figure 9A and Figure 9B.

**Table 2: Temperature Stability Test Results**

| | 4°C (mg/mL) | 25°C (mg/mL) | 37°C (mg/mL) | 50°C (mg/mL) | 60°C (mg/mL) | 70°C (mg/mL) | 80°C (mg/mL) | 90°C (mg/mL) |
|---|---|---|---|---|---|---|---|---|
| T002G-A | 0.992 | 1.082 | 1.083 | 0.219 | 0.012 | 0.008 | 0.019 | 0.046 |
| T002G-B | 1.007 | 1.035 | 1.043 | 0.023 | 0.007 | 0.01 | 0.018 | 0.029 |
| T002G-C | 0.987 | 1 | 1.011 | 0.077 | 0.01 | 0.023 | 0.012 | 0.041 |
| T002G-D | 1.029 | 0.995 | 0.988 | 0.18 | 0.12 | 0.085 | 0.018 | 0.091 |
| T002G-E | 1.048 | 1.056 | 1.034 | 0.525 | 0.026 | 0.064 | 0.012 | 0.026 |
| T002G-F | 1.081 | 1.071 | 0.986 | 0.997 | 0.96 | 0.45 | 0.043 | 0.043 |
| T002G-G | 0.998 | 0.989 | 1.011 | 0.99 | 0.99 | 0.34 | 0.059 | 0.033 |
| T002G-H | 0.952 | 0.956 | 0.952 | 0.178 | 0.083 | 0.051 | 0.052 | 0.034 |
| T002G-I | 0.982 | 1.045 | 1.015 | 0.152 | 0.083 | 0.056 | 0.045 | 0.043 |
| T002G-J | 0.994 | 0.992 | 1.007 | 0.987 | 1.011 | 0.385 | 0.015 | 0.051 |
| T002G-K | 0.997 | 1.023 | 1.027 | 1.015 | 1.017 | 0.343 | 0.041 | 0.029 |
| NbHSA | 1.012 | 1.069 | 1.041 | 0.341 | 0.198 | 0.197 | 0.049 | 0.054 |

The results indicate that the mon-ECD-binding molecule T002G-A has moderate temperature stability, remaining stable at temperatures below 37°C but suffering substantial protein loss at 50°C. Among the dual-ECD-binding fusion proteins, PTH(15-34), PTHrP(15-36), and different linkers do not significantly improve the temperature stability of the fusion proteins. However, the addition of VHH22A3, the nanobody of PTHrP, significantly enhances the temperature stability of the fusion proteins, regardless of being fused to the front-end (T002G-F) or rear-end of NbHSA. This may be related to the high inherent stability of nanobody, which consequently improves the stability of the smaller PTH(1-34) molecule after fusion expression.

Furthermore, the test results of the triple-ECD-binding fusion proteins T002G-J and T002G-K show that the addition of VHH22A3 (the nanobody of PTHrP) enhances the temperature stability of the fusion protein molecules. Whether fused to the front-end (T002G-J) or rear-end (T002G-K) of the human serum protein nanobody NbHSA, the fusion proteins exhibit robust temperature stability, with almost no protein loss observed after incubation at 60°C for 4 hours.

### Test Example 4: Freeze-Thaw Stability Study

The freeze-thaw stability of the fusion proteins prepared in Examples 1-11 was determined according to the following method.

The stock solutions of the proteins prepared in Examples 1-11 were separately diluted to 1 mg/mL with the corresponding buffer and filtered through a 0.22 µm filter membrane. Each diluted solution was then divided into 3 aliquots, and then subjected to freeze-thaw cycles in a -80°C refrigerator. Each freezing step lasted for 30 minutes, followed by natural thawing at room temperature. Samples were taken at the 0th, 1st, 3rd, and 5th freeze-thaw cycles to observe the properties and measure the OD value at a wavelength of 280 nm.

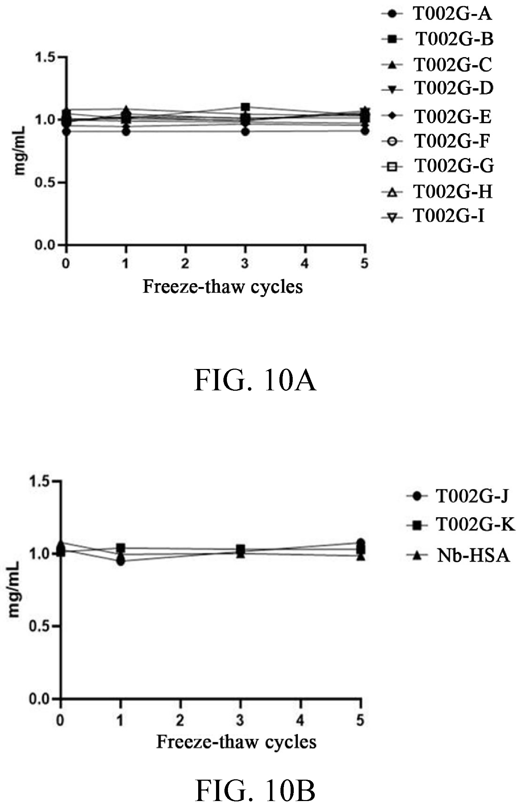

The test results are shown in Table 3, Figure 10A and Figure 10B.

**Table 3: Freeze-Thaw Stability Test Results**

| | 0^{th} cycle (mg/mL) | 1^{st} cycle (mg/mL) | 3^{rd} cycle (mg/mL) | 5^{th} cycle (mg/mL) |
|---|---|---|---|---|
| T002G-A | 0.906 | 0.905 | 0.906 | 0.911 |
| T002G-B | 1.048 | 1.009 | 1.102 | 1.036 |
| T002G-C | 1.081 | 1.085 | 1.044 | 1.031 |
| T002G-D | 0.998 | 0.989 | 0.982 | 0.972 |
| T002G-E | 0.952 | 0.945 | 0.966 | 0.956 |
| T002G-F | 0.982 | 1.046 | 1.008 | 1.046 |
| T002G-G | 0.992 | 1.024 | 1.015 | 1.015 |
| T002G-H | 1.007 | 1.006 | 0.996 | 1.071 |
| T002G-I | 0.987 | 1.021 | 0.992 | 1.057 |
| T002G-J | 1.029 | 0.949 | 1.015 | 1.075 |
| T002G-K | 1.027 | 1.043 | 1.035 | 1.024 |
| NbHSA | 1.078 | 0.995 | 1.002 | 0.985 |

The results show that the fusion proteins T002G-A to T002G-K maintain the stability of the protein stock solution during repeated freeze-thaw cycles from -80°C to room temperature, without protein loss or precipitation formation, indicating that they have excellent freeze-thaw stability.

### Test Example 5: Protein Solubility Study

Two recombinant proteins (T002G-J and T002G-K) were selected to determine their solubility according to the following method.

The stock solutions of the proteins prepared in Examples 10-11 were separately diluted to 1 mg/mL with the corresponding buffer. A 150 mL aliquot of each diluted solution was subjected to centrifugation using a Sartorius ultrafiltration concentration tube with 3000D cut-off aperture at a centrifugal force of 3000 g. The protein concentration and appearance (checking for the presence of solid precipitates) were determined when the volume was 30 mL, 15 mL, 10 mL, 5 mL, 3 mL, and 1.5 mL, respectively.

The test results are shown in Table 4 and Figure 10B.

**Table 4: Solubility Test Data of Fusion Proteins**

| | Volume (mL) | Concentration (mg/mL) | Appearance |
|---|---|---|---|
| T002G-J | 30 | 5.01 | Clear (no precipitate) |
| | 15 | 10.21 | Clear (no precipitate) |
| | 10 | 14.87 | Clear (no precipitate) |
| | 5 | 29.66 | Clear (no precipitate) |
| | 3 | 48.81 | Clear (no precipitate) |
| | 1.5 | 98.01 | Clear (no precipitate) |
| T002G-K | 30 | 5.21 | Clear (no precipitate) |
| | 15 | 9.98 | Clear (no precipitate) |
| | 10 | 14.89 | Clear (no precipitate) |
| | 5 | 28.98 | Clear (no precipitate) |
| | 3 | 49.01 | Clear (no precipitate) |
| | 1.5 | 98.32 | Clear (no precipitate) |

The results indicate that both T002G-J and T002G-K have good solubility, remaining clear without precipitate formation even when the concentration reaches approximately 100 mg/mL.

The embodiments of the present disclosure have been described in detail above. However, the present disclosure is not limited to the above embodiments. Various modifications can be made without departing from the gist of the present disclosure within the scope of knowledge possessed by those of ordinary skill in the art. Furthermore, the embodiments of the present disclosure and the features thereof can be combined with each other without conflict.

## Claims

1. A recombinant parathyroid hormone (PTH) fusion protein, comprising:
A1) at least one signaling domain molecule capable of activating a parathyroid hormone receptor 1 (PTHR1) intracellular pathway;
A2) one or more molecules capable of binding to an extracellular domain (ECD) of PTHR1; and
A3) a human serum albumin nanobody;
wherein an amino acid sequence of the human serum albumin nanobody is shown as SEQ ID NO:7.

2. The recombinant PTH fusion protein according to claim 1, wherein an amino acid sequence of the signaling domain molecule capable of activating the PTHR1 intracellular pathway comprises a sequence shown as SEQ ID NO: 1;
preferably, the signaling domain molecule capable of activating the PTHR1 intracellular pathway is at least one of PTH(1-14) to PTH(1-34).

3. The recombinant PTH fusion protein according to claim 1 or 2, wherein an amino acid sequence of the molecule capable of binding to the ECD of PTHR1 comprises at least one of a sequences shown as SEQ ID NO: 3, SEQ ID NO: 5, or SEQ ID NO: 6.

4. The recombinant PTH fusion protein according to claim 1 or 2, wherein the molecule capable of binding to the ECD of PTHR1 is at least one selected from the group consisting of PTH(15-34), PTH(14-34), PTH(13-34), PTH(12-34), PTH(11-34), PTH(10-34), PTH(9-34), PTH(8-34), PTH(7-34), PTH(6-34), PTH(5-34), PTH(4-34), PTH(2-34), PTH(15-84), PTH(14-84), PTH(13-84), PTH(12-84), PTH(11-84), PTH(10-84), PTH(9-84), PTH(8-84), PTH(7-84), PTH(6-84), PTH(5-84), PTH(4-84), PTH(3-84), PTH(2-84), PTHrP(15-36), PTHrP(14-36), PTHrP(13-36), PTHrP(12-36), PTHrP(11-36), PTHrP(10-36), PTHrP(9-36), PTHrP(8-36), PTHrP(7-36), PTHrP(6-36), PTHrP(5-36), PTHrP(4-36), PTHrP(3-36), PTHrP(2-36), and a nanobody of PTHR1.

5. The recombinant PTH fusion protein according to claim 1, wherein the signaling domain molecule capable of activating the PTHR1 intracellular pathway, the molecule capable of binding to the ECD of PTHR1 or the human serum albumin nanobody of the recombinant PTH fusion protein are linked via a linker;
preferably, the linker comprises an amino acid sequence as shown in any one of SEQ ID NO: 8 to SEQ ID NO: 10.

6. A method for constructing the recombinant PTH fusion protein according to any one of claims 1 to 5, comprising introducing an expression vector containing an encoding gene of the recombinant PTH fusion protein of any one of claims 1 to 5 into a host cell to express the encoding gene, and performing separation and purification to obtain the recombinant PTH fusion protein.

7. A biomaterial, wherein the recombinant PTH fusion is any one of B1) to B4):
B1) a nucleic acid molecule encoding the recombinant PTH fusion protein according to any one of claims 1 to 5;
B2) an expression cassette containing the nucleic acid molecule described in B1);
B3) a recombinant vector containing the nucleic acid molecule described in B1) or the expression cassette described in B2); and
B4) a host cell containing the nucleic acid molecule described in B1), the expression cassette described in B2), or the recombinant vector described in B3).

8. Use of the recombinant PTH fusion protein of any one of claims 1 to 5 in preparing a product for the treatment and/or prevention of hypoparathyroidism.

9. A drug comprising the recombinant PTH fusion protein of any one of claims 1 to 5 and a pharmaceutically acceptable excipient.

10. A combination drug comprising at least one of the recombinant PTH fusion protein of any one of claims 1 to 5 and the drug of claim 9, and an anti-tumor targeting drug.
